Europäisches Patentamt

European Patent Office

Office européen des brevets

(11) Veröffentlichungsnummer: **0 121 109**
**B1**

# (12) EUROPÄISCHE PATENTSCHRIFT

(45) Veröffentlichungstag der Patentschrift: **13.06.90**

(21) Anmeldenummer: **84102206.4**

(22) Anmeldetag: **02.03.84**

(51) Int. Cl.⁵: **B 01 D 53/34, B 01 D 53/14**

(54) Verfahren zum Entfernen von C02 und/oder H2S aus Gasen.

(30) Priorität: **08.03.83 DE 3308088**

(43) Veröffentlichungstag der Anmeldung:
**10.10.84 Patentblatt 84/41**

(45) Bekanntmachung des Hinweises auf die
Patenterteilung:
**13.06.90 Patentblatt 90/24**

(84) Benannte Vertragsstaaten:
**AT BE DE FR GB IT NL SE**

(56) Entgegenhaltungen:
**EP-A-0 107 783**
**DD-A- 119 610**
**DE-A-2 551 717**

(73) Patentinhaber: **BASF Aktiengesellschaft**
**Carl-Bosch-Strasse 38**
**D-6700 Ludwigshafen (DE)**

(72) Erfinder: **Wagner, Eckhart, Dr.**
**Birkenstrasse 21a**
**D-6701 Maxdorf (DE)**
Erfinder: **Volkamer, Klaus, Dr.**
**Heidelberger Ring 21**
**D-6710 Frankenthal (DE)**
Erfinder: **Hefner, Werner, Dr.**
**Danziger Strasse 1c**
**D-6840 Lampertheim (DE)**
Erfinder: **Wagner, Ulrich, Dr.**
**Knospstrasse 7**
**D-6703 Limburgerhof (DE)**

Courier Press, Leamington Spa, England.

## Beschreibung

Die vorliegende Erfindung betrifft ein Verfahren zum Entfernen von $CO_2$ und/oder $H_2S$ aus Gasen mittels einer wäßrigen Absorptionsflüssigkeit.

Es ist bekannt, z.B. aus A.L. Kohl—F.C. Riesenfeld, Gas- Purification, 3. Auflage, 1979, wäßrige Lösungen von Monoethanolamin oder Diethanolamin oder Mischungen von Cyclotetramethylensulfon und einer wäßrigen Lösung von Diisopropanolamin als Lösungsmittel zur Entfernung von $CO_2$ und/oder $H_2S$ aus Gasen zu verwenden. Bei diesen Verfahren ist es erforderlich, das mit $CO_2$ und gegebenenfalls $H_2S$ beladene Lösungsmittel in einer Ausstreifkolonne durch Zufuhr von Dampf zu regenerieren, was mit erheblichem Energieaufwand verbunden ist. Bei der Entfernung von $CO_2$ und gegebenenfalls $H_2S$ aus höhere Kohlenwasserstoffe enthaltenden Erdgasen mittels einer Mischung von Cyclotetramethylensulfon und einer wäßrigen Lösung von Diisopropanolamin kommt als zusätzlicher Nachteil hinzu, daß die höheren Kohlenwasserstoffe in diesem Lösungsmittel eine relativ hohe Löslichkeit aufweisen, so daß das am Kopf der Ausstreifkolonne abgezogene Sauergas einen relativ hohen Gehalt an Kohlenwasserstoffen aufweist, der, falls das Sauergas $H_2S$ enthält, zu Schwierigkeiten in einer nachgeschalteten Claus-Anlage führen kann. Primäre oder sekundäre Alkanolamine, wie Monoethanolamin oder Diethanolamin, können außerdem in der Regel nur als wäßrige Lösungen mit verhältnismäßig geringer Konzentration an diesen Alkanolaminen verwendet werden, da bei Verwendung höherer Konzentrationen schwere Korrosionsschäden an Anlageteilen auftreten können.

In der DE—A—2 551 717 wird weiter ein Verfahren zum Auswaschen von $CO_2$ und/oder $H_2S$ aus Gasen mit chemischen oder physikalischen Lösungsmitteln beschrieben, bei dem das aus einer zweistufigen Absorption erhaltene, mit $CO_2$ und/oder $H_2S$ beladene Lösungsmittel, z.B. eine Methyldiethanolamin enthaltende wäßrige Absorptionsflüssigkeit, in zwei Entspannungsstufen bei Drucken oberhalb von Atmosphärendruck entspannt und dabei teilweise regeneriert wird. Anschließend wird ein Teilstrom des entspannten Lösungsmittels in einem Ausstreifer weiter regeneriert. Die ganz und teilweise regenerierten Lösungsmittel werden in die Absorption zurückgeführt. Das Verfahren zeichnet sich zwar durch eine hohe Wirtschaftlichkeit aus, ist jedoch noch nicht für alle Anwendungsfälle zufriedenstellend.

Es bestand daher Bedarf nach einem Verfahren zum Entfernen von $CO_2$ und/oder $H_2S$ aus Gasen, bei dem die Nachteile der bekannten Verfahren vermieden werden können.

Es wurde nun ein vorteilhaftes Verfahren gefunden zum Entfernen von $CO_2$ und/oder $H_2S$ aus $CO_2$ und/oder $H_2S$ enthaltenden Gasen, bei dem man

a) die $CO_2$ und/oder $H_2S$ enthaltenden Gase in einer Absorptionsstufe bei Temperaturen von 40 bis 100°C mit einer 20 bis 70 Gew.-% Methyldiethanolamin enthaltenden wäßrigen Absorbtionsflüssigkeit behandelt,

b) am Kopf der Absorptionsstufe die behandelten Gase abzieht,

c) am Sumpf der Absorptionsstufe die mit $CO_2$ und/oder $H_2S$ beladene wäßrige Absorptionsflüssigkeit abzieht und zur Regenerierung in mindestens zwei Entspannungsstufen entspannt und dabei die letzte Entspannungsstufe bei vermindertem Druck gegenüber Atmosphärendruck betreibt.

d) zum Ausgleich der Wasserverluste durch in den am Kopf der Absorptionsstufe und den Entspannungsstufen abgezogenen Gasströmen enthaltenes Wasser am Sumpf der vorletzten Entspannungsstufe eine dem Wasserverlust entsprechende Menge Wasserdampf zuführt und

e) die regenerierte Absorptionsflüssigkeit in die Absorptionsstufe zurückführt.

Nach dem neuen Verfahren wird das mit $CO_2$ und/oder $H_2S$ beladene Lösungsmittel ohne Verwendung eine Ausstreifkolonne allein durch Entspannung regeneriert, so daß eine erhebliche Reduktion sowohl der Investitionskosten als auch der Energieverbrauchskosten erreicht wird. Weiter können bei dem neuen Verfahren relativ hohe Methyldiethanolaminkonzentrationen in der Absorptionsflüssigkeit verwendet werden, ohne daß es zu Korrosionsschäden in der Gaswaschanlage kommt. Ein weiterer Vorteil des Verfahrens besteht darin, daß Wasserverluste, die im Gaswaschanlagen durch in den am Kopf der Absorptionskolonne und der Entspannungsbehälter abgezogenen Gasströmen enthaltenes Wasser entstehen, durch Zuführung einer dem Wasserverlust entsprechenden Menge Wasserdampf am Sumpf der vorletzten Entspannungsstufe ausgeglichen werden können. Durch diese Arbeitsweise kann neben der Regelung des Wasserhaushaltes der Gaswaschanlage gleichzeitig der Wärmehaushalt der Gaswaschanlage geregelt werden, so daß ein für die Regelung des Wärmehaushaltes in der Gaswaschanlage vorhandener Wärmetauscher kleiner ausgeführt werden oder gegebenenfalls ganz wegfallen kann. Durch das Betreiben der letzten Entspannungsstufe bei vermindertem Druck wird eine regenerierte Absorptionsflüssigkeit mit einem geringerem $CO_2$- und/oder $H_2S$-Gehalt erhalten, so daß geringere Mengen an Absorptionsflüssigkeit im Kreis geführt werden müssen, wodurch sich entsprechende Einsparungen bei den Energieverbräuchen ergeben.

In einer weiteren bevorzugten Ausführungsform des Verfahrens wird der verminderte Druck in der letzten Entspannungsstufe mittels eines Wasserdampfstrahlers erzeugt, wobei man vorteilhaft das am Kopf der letzten Entspannungsstufe abgezogene Gas zusammen mit dem Wasserdampf, mit dem der Wasserdampfstrahler betrieben wird, am Sumpf der vorletzten Entspannungsstufe zuführt.

Als nach dem erfindungsgemäßen Verfahren zu behandelnde Gase kommen beispielsweise Kohlevergasungsgase, Synthesegase, Koksofengase und vorzugsweise Erdgase in Betracht. Vorteilhaft wird das Verfahren zum Entfernen von $CO_2$ und/oder $H_2S$ aus Erdgasen verwendet, die neben Methan noch höhere Kohlenwasserstoffe enthalten. Bei diesen höheren Kohlenwasserstoffen handelt es sich im allgemeinen um $C_2$- bis $C_{30}$-Kohlenwasserstoffe, vorzugsweise $C_2$- bis $C_{20}$-Kohlenwasserstoffe, insbesondere $C_2$- bis $C_{12}$-Kohlenwasserstoffe, die in der Regel aliphatisch sind. z.B. Ethan, Propan, Isobutan, n-Butan, Isopentan, n-Pentan, die Hexane, Heptane, Octane, Nonane, Decane und die höheren Homologen. Die höhere Kohlenwasserstoffe können neben den aliphatischen Kohlenwasserstoffen auch noch aromatische Kohlenwasserstoffe wie Benzol enthalten. Im allgemeinen beträgt der Gehalt der Erdgase an den höheren Kohlenwasserstoffen 0,1 bis 40 Mol.%, vorzugsweise 0,5 bis 30 Mol%, insbesondere 1 bis 20 Mol%.

Die Gase weisen im allgemeinen einen $CO_2$-Gehalt von 1 bis 90 Mol.%, vorzugsweise 2 bis 90 Mol.%, insbesondere 5 bis 60 Mol.%, auf. Neben dem $CO_2$ können die Gase als weiteres Sauergas $H_2S$ enthalten oder sie können $H_2S$ allein enthalten, z.B. in Mengen von wenigen Mol.-ppm, beispielsweise 1 Mol.-ppm bis 50 Mol.%, vorzugsweise 10 Mol.-ppm bis 40 Mol.%.

Als Lösungsmittel wird für das erfindungsgemäße Verfahren eine 20 bis 70 Gew.%, vorzugsweise 30 bis 65 Gew.%, insbesondere 40 bis 60 Gew.% Methyldiethanolamin enthaltende wäßrige Absorptionsflüssigkeit verwendet. Zweckmäßig wird eine wäßrige Methyldiethanolaminlösung eingesetzt, z.B. eine wäßrige Lösung von Methyldiethanolamin technischer Reinheit. In einer vorteilhaften Ausführungsform des Verfahrens verwendet man eine wäßrige Methyldiethanolaminlösung, die zusätzlich 0,1 bis 1 Mol/l, insbesondere 0,2 bis 0,8 Mol/l, insbesondere 0,25 bis 0,6 Mol/l eines primären Amins oder Alkanolamins wie Monoethanolamin, vorzugsweise eines sekundären Amins oder Alkanolamins, vorteilhaft Methylmonoethanolamin, ganz besonders vorteilhaft Piperazin, enthält. Das erfindungsgemäße Verfahren wird in der Weise durchgeführt, daß das $CO_2$ und/oder $H_2S$ enthaltende Gas zunächst in einer Absorptionsstufe mit der Methyldiethanolamin enthaltenden Absorptionsflüssigkeit behandelt wird. Dabei werden in der Absorptionsstufe Temperaturen von 40 bis 100°C, vorzugsweise 50 bis 90°C, insbesondere 60 bis 80°C für die Absorptionsflüssigkeit aufrechterhalten. Im allgemeinen werden in der Absorptionsstufe Drucke von 10 bis 110 bar, vorzugsweise 20 bis 100 bar, insbesondere 30 bis 90 bar angewendet. Als Absorptionsstufe wird zweckmäßig eine Absorptionskolonne verwendet, im allgemeinen eine Füllkörperkolonne oder eine mit Böden ausgestattete Kolonne. Zweckmäßig wird das zu behandelnde Gas am Sumpf und die Absorptionsflüssigkeit am Kopf der Absorptionskolonne zugeführt, wobei die sauren Gase $CO_2$ und/oder $H_2S$ im Gegenstrom ausgewaschen werden. Während gegebenenfalls vorhandenes $H_2S$ zweckmäßig weitgehend, im allgemeinen auf $H_2S$-Gehalte im behandelten Gas von höchstens 120 Mol.-ppm, vorzugsweise höchstens 10 Mol.-ppm, insbesondere höchstens 3 Mol.-ppm ausgewaschen wird, kann es vorteilhaft sein, das $CO_2$ im Gas soweit auszuwaschen, daß die $CO_2$-Gehalte im

behandelten Gas höchstens etwa 0,5 bis 6, vorzugsweise 0,5 bis 5, insbesondere 1 bis 4 Mol.% betragen. Das behandelte Gas wird zweckmäßig am Kopf der Absorptionsstufe, zweckmäßig an einem Punkt, der oberhalb der Zuführung der Absorptionsflüssigkeit liegt, abgezogen. Die mit den Sauergasen $CO_2$ und/oder $H_2S$ beladene Absorptionsflüssigkeit wird zweckmäßig am Sumpf der Absorptionszone abgezogen.

Anschließend wird die beladene Absorptionsflüssigkeit in mindestens 2, zweckmäßig 2 bis 5, vorzugsweise 2 oder 3, Entspannungsstufen regeneriert, wobei die letzte Entspannungsstufe bei vermindertem Druck gegen- über Atmosphärendruck betrieben wird und gegebenenfalls gleichzeitig zum Ausgleich der Wasserverluste durch in den am Kopf der Absorptionsstufe und den Entspannungsstufen abgezogenen Gasströmen enthaltenes Wasser am Sumpf der vorletzten Entspannungsstufe eine dem Wasserverlust entsprechende Menge Wasserdampf zugeführt wird. Vorzugsweise wird dabei in der letzten Entspannungsstufe ein Druck von 0,5 bis etwa 1 bar, vorzugsweise 0,7 bis etwa 1 bar aufrechterhalten. Der verminderte Druck kann in der letzten Entspannungsstufe beispielsweise unter Verwendung eines zur Vakuumerzeugung üblicherweise verwendeten Apparates, z.B. eine Vakuumpumpe, eingestellt werden. Mit besonderem Vorteil wird der verminderte Druck in der letzten Entspannungsstufe mittels eines Wasserdampfstrahlers erzeugt.

Zum Ausgleich der Wasserverluste, die bei dem Verfahren durch in den am Kopf der Absorptionsstufe und den Entspannungsstufen abgezogenen Gasströmen enthaltenes Wasser entstehen, wird zweckmäßig am Sumpf der vorletzten Entspannungsstufe eine dem Wasserverlust entsprechende Menge Wasserdampf zugeführt. In der Regel wird das in den abgezogenen Gasströmen enthaltene Wasser im wesentlichen als Wasserdampf abgezogen. Dem Sumpf der vorletzten Entspannungsstufe kann Niederdruck-, Mitteldruck- oder Hochdruckdampf, d.h. z.B. 1,5- bis 100-bar-Dampf zugeführt werden. Vorzugsweise verwendet man Dampf im niederen Druckbereich, z.B. 1,5- bis 10-bar-Dampf, vorteilhaft, 1,5- bis 5-bar-Dampf, da dieser Niederdruckdampf im allgemeinen wohlfeil zur Verfügung steht.

Das am Kopf der letzten Entspannungsstufe angezogene Gas kann an die Atmosphäre abgegeben werden oder, falls es noch $H_2S$ enthält, durch Oxidation des $H_2S$, z.B. in eienr Claus-Anlage, aufgearbeitet werden. In einer vorteilhaften Ausführungsform des Verfahrens wird der verminderte Druck in der letzten Entspannungsstufe mittels eines Wasserdampfstrahlers erzeugt und dabei zweckmäßig das am Kopf der letzten Entspannungsstufe abgezogene Gas zusammen mit dem Wasserdampf, mit dem der Wasserdampfstrahler betrieben wird, am Sumpf der vorletzten Entspannungsstufe zugeführt.

Falls der Wasserdampf zum Betrieb des Wasserdampfstrahlers am Sumpf der vorletzten Entspannungsstufe zugeführt wird, wird der Wasserdampfstrahler zweckmäßig mit einer solchen Menge Wasserdampf betrieben, wie sie zum Ausgleich der Wasserverluste des Verfahrens erforderlich ist. Es ist jedoch auch möglich, den Wasserdampfstrahler mit einer geringeren als zum Ausgleich der Wasserverluste entsprechenden Wasserdampfmenge zu betreiben und am Sumpf der vorletzten Entspannungsstufe zusätzlich die zum Ausgleich der Wasserverluste noch fehlende Wasserdampfmenge zuzuführen. Zum Betrieb des Wasserdampfstrahlers kann Mitteldruck- oder Hochdruckdampf verwendet werden. Verzugsweise verwendet man Dampf in mittleren Druckbereich, z.B. 5- bis 20-bar-Dampf, vorzugsweise 5- bis 10-bar-Dampf.

Die vorletzte Entspannungsstufe wird zweckmäßig bei einem Druck von etwa 1 bis 5 bar, vorzugsweise 1 bis 2,5 bar, insbesondere 1 bis 1,5 bar betrieben.

Für die Entspannung werden zweckmäßig Entspannungsbehälter, die z.B. auch als Kolonnen gestaltet sein können, verwendet. Diese Entspannungsbehälter können frei von besonderen Einbauten sein. Es können jedoch auch mit Einbauten, z.B. mit Füllkörpern ausgestattete Kolonnen verwendet werden.

Die Sauergase $CO_2$ und/oder $H_2S$ werden zweckmäßig am Kopf der letzten Entspannungsstufe bzw., falls das am Kopf der letzten Entspannungsstufe abgezogene Gas zusammen mit dem Wasserdampf für den Betrieb des Wasserdampfstrahlers am Sumpf der vorletzten Entspannungsstufe zugeführt wird, am Kopf der vorletzten Entspannungsstufe abgezogen. Die am Sumpf der letzten Entspannungsstufe abgezogene regenerierte Absorptionsflüssigkeit wird in die Absorptionsstufe zurückgeführt.

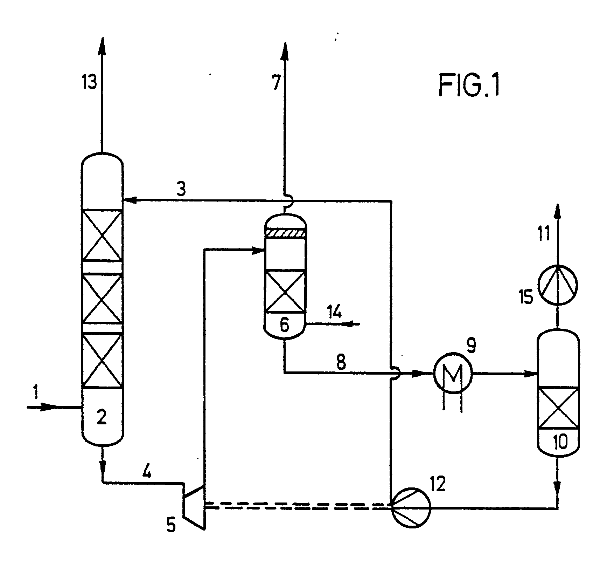

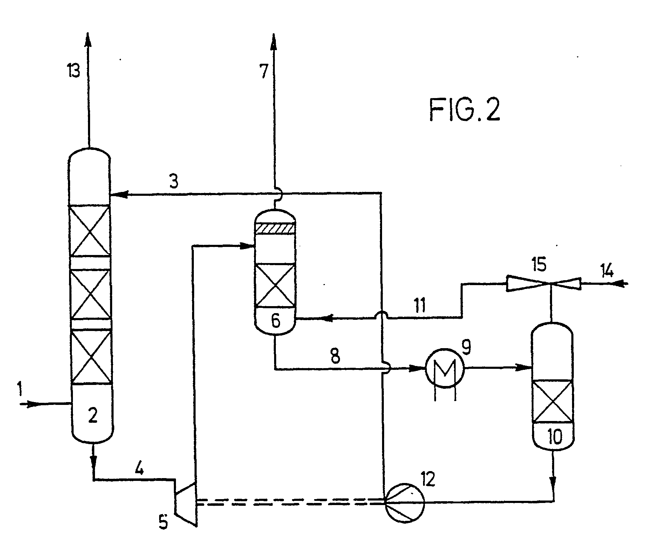

Nachfolgend werden weitere Einzelheiten der Erfindung anhand von zwei Ausführungsbeispielen, deren Verfahrensablauf in den Figuren 1 und 2 schematisch dargestellt ist, erläutert.

Gemäß Figur 1 wird ein $CO_2$ und/oder $H_2S$ enthaltendes Gas, z.B. ein höhere Kohlenwasserstoffe, z.B. aliphatische $C_2$- bis $C_{10}$-Kohlenwasserstoffe enthaltendes Erdgas, über Leitung 1 unter Druck in dem Sumpf der Absorptionskolonne 2 gegeben. Gleichzeitig wird über Leitung 3 als Absorptionsflüssigkeit 20 bis 70 gew.%ige wäßrige Methyldiethanolaminlösung auf dem Kopf der Absorptionskolonne gegeben. Die Absorptionsflüssigkeit, die im Gegenstrom zum Gas geführt wird, belädt sich mit den sauren Gasen $CO_2$ und/oder $H_2S$, und die beladene Absorptionsflüssigkeit wird über Leitung 4 am Sumpf der Absorptionskolonne abgezogen. Das gewaschene Gas wird über Leitung 13 am Kopf der Absorptionskolonne abgezogen. Der beladene Absorptionsflüssigkeitsstrom 4 wird anschließend in einen Entspannungsverdampfungsbehälter 6 entspannt, beispielsweise über ein Ventil oder vorzugsweise über eine Entspannungsturbine 5. Hierbei wird ein Kohlenwasserstoffe und $CO_2$ enthaltendes Zwischenentspannungsgas aus der Absorptionsflüssigkeit freigesetzt, das über Leitung 7 am Kopf des Entspannungsbehälters 6 abgezogen wird. In den Sumpf des Entspannungsbehälters 6 wird gegebenenfalls zum Ausgleich der Wasserverluste des Systems über Leitung 14 Wasserdampf, z.B. 2,5 bar-Niederdruckdampf, eingeleitet. Am Boden des Entspannungsbehälters 6 wird die teilentspannte Absorptionsflüssigkeit über Leitung 8 abgezogen, in Wärmetauscher 9 erwärmt, z.B. um 1 bis 15°C, und die erwärmte Absorptionsflüssigkeit wird in einem zweiten Entspannungsbehälter 10 entspannt, in dem z.B. mittels der Vakuumpumpe 15 ein Druck von z.B. 0,5 bar bis weniger als Atmosphärendruck aufrecht erhalten wird. Hierbei wird ein $CO_2$-reiches Entspannungsgas, z.B. mit einer $CO_2$-Konzentration von 98 Mol.%, freigesetzt, das über Leitung 11 am Kopf des Entspannungsbehälters 10 abgezogen wird. Die am Sumpf des Entspannungsbehälters 10 abgezogene regenerierte Absorptionsflüssigkeit wird mit Hilfe einer Umlaufpumpe 12 zum Kopf der Absorptionskolonne 2 zurückgeführt.

In einem weiteren beispielhaften Ausführungsbeispiel (vgl. Fig. 2) wird wie im ersten Ausführungsbeispiel verfahren, wobei jedoch anstelle einer Vakuumpumpe der Dampfstrahler 15 zur Erzeugung des verminderten Drucks im zweiten Entspannungsbehälter 10 verwendet wird, dem über Leitung 14 z.B. eine solche Menge Wasserdampf zugeführt wird, wie sie zum Ausgleich der Wasserverluste des Systems erforderlich ist. Das am Kopf des Entspannungsbehälters 10 abgezogene Gas wird zusammen mit dem Wasserdampf, mit dem der Wasserdampfstrahler 15 betrieben wird, am Sumpf des ersten Entspannungsbehälters 6 zugeführt.

Das folgende Beispiel veranschaulicht die Erfindung.

Beispiel

Man verwendet eine Gaswaschanlage, in der sich anschließend an eine Absorptionskolonne drei hintereinandergeschaltete Entspannungsbehälter befinden. In der Absorptionskolonne werden 3,15 kmol/h eines $CO_2$-haltigen Erdgases mit einer 50 gew.%igen wäßrigen Methyldiethanolamin-Lösung als Absorptionsflüssigkeit bei einem Druck von 75 bar gewaschen.

Das zu reinigende Gas hat folgende Zusammensetzung:

| | |
|---|---|
| $CO_2$ | 10,0 Mol.% |
| $CH_4$ | 75,0 Mol.% |
| höhere Kohlenwasserstoffe ($C_2$- bis $C_{12}$-Kohlenwasserstoffe) | 15,0 Mol.% |

Dem Temperatur des Absorptionsmittels im Zulauf zur Absorptionskolonne beträgt 70°C. Der $CO_2$-Gehalt im gewaschenen Gas beträgt weniger als 2 Mol.%. Das die Absorptionskolonne verlassende beladene Waschmittel wird in einem ersten Entspannungsbehälter auf einen Druck von 30 bar entspannt. Hierbei werden 6,6 mol/h eines kohlenwasserstoffreichen Zwischenentspannungsgases mit einer $CO_2$-Konzentration von weniger als 10 Mol.% aus der Lösung freigesetzt und am Kopf des ersten Entspannungsbehälters abgezogen. Die teilentspannte Absorptionsflüssigkeit wird anschließend in einem Wärmetauscher erwärmt. Die erwärmte Absorptionsflüssigkeit wird in einem zweiten Entspannungsbehälter auf 1,5 bar entspannt. Hierbei werden 0,13 kmol/h eines $CO_2$-reichen Entspannungsgases mit einer $CO_2$-Konzentration von mehr als 98 Mol.% frei, das am Kopf des zweiten Entspannungsbehälters abgezogen wird.

Die am Boden des zweiten Entspannungsbehälters abgezogene Absorptionsflüssigkeit wird schließlich nach Durchlaufen eines Wärmetauschers in einen dritten Entspannungsbehälter entspannt, in dem mittels eines Wasserdampfstrahlers ein Druck von 0,8 bar aufrechterhalten wird Das am Kopf des dritten Entspannungsbehälters abgezogene Gas wird zusammen mit dem Wasserdampf, mit dem der Wasserdampfstrahler betrieben wird, in den Sumpf des zweiten Entspannungsbehälters eingeleitet. Die am Sumpf des dritten Entspannungsbehälters abgezogene Absorptionsflüssigkeit wird mit Hilfe einer Umlaufpumpe zum Kopf der Absorptionskolonne zurückgepumpt.

**Patentansprüche**

1. Verfahren zum Entfernen von $CO_2$ und/oder $H_2S$ aus $CO_2$ und/oder $H_2S$ enthaltenden Gasen, bei dem man

a) die $CO_2$ und/oder $H_2S$ enthaltenden Gase in einer Absorptionsstufe bei Temperaturen von 40 bis 100°C mit einer 20 bis 70 Gew.-% Methyldiethanolamin enthaltenden wäßrigen Absorptionsflüssigkeit behandelt,

b) am Kopf der Absorptionsstufe die behandelten Gase abzieht,

c) am Sumpf der Absorptionsstufe die mit $CO_2$ und/oder $H_2S$ beladene wäßrige Absorptionsflüssigkeit abzieht und zur Regenerierung in mindestens zwei Entspannungsstufen entspannt und dabei die letzte Entspannungsstufe bei vermindertem Druck gegenüber Atmosphärendruck betreibt,

d) zum Ausgleich der Wasserverluste durch in den am Kopf der Absorptionsstufe und den Entspannungsstufen abgezogenen Gasströmen enthaltenes Wasser am Sumpf der vorletzten Entspannungsstufe eine dem Wasserverlust entsprechende Menge Wasserdampf zuführt und

e) die regenerierte Absorptionsflüssigkeit in die Absorptionsstufe zurückführt.

2. Verfahren nach Anspruch 1, dadurch gekennzeichnet, daß der verminderte Druck in der letzten Entspannungsstufe mittels eines Wasserdampfstrahlers erzeugt wird und das am Kopf der letzten Entspannungsstufe abgezogene Gas zusammen mit dem Wasserdampf, mit dem der Wasserdampfstrahler betrieben wird, am Sumpf der vorletzten Entspannungsstufe zugeführt wird.

**Revendications**

1. Procédé pour éliminer le $CO_2$ et/ou l'$H_2S$ de gaz qui contiennent du $CO_2$ ou de l'$H_2S$, dans lequel

a) on traite les gaz contenant du $CO_2$ et/ou de l'$H_2S$ dans un étage d'absorption, à des températures de 40 à 100°C, avec un liquide d'absorption aqueux contenant 20 à 70% en poids de méthyldiéthanolamine,

b) on extrait les gaz traités en tête de l'étage d'absorption,

c) on extrait le liquide d'absorption aqueux chargé de $CO_2$ et/ou d'$H_2S$ en bas de l'étage d'absorption et, pour sa régéneration, on le détend dans deux étages de détente au moins, le dernier étage de détente étant exploité sous pression réduite par rapport à la pression atmosphérique,

d) pour compenser les pertes d'eau dues à l'eau contenue dans les courants de gaz extraits en tête de l'étage d'absorption et dans les étages de détente, on envoie, en bas de l'avant-dernier étage de détente, une quantité de vapeur d'eau qui correspond à la perte d'eau et

e) on renvoie dans l'étage d'absorption le liquide d'absorptions régénéré.

2. Procédé selon la revendication 1, caractérisé en ce que la pression réduite dans le dernier étage de détente est produite au moyen d'une trompe à vapeur d'eau et le gaz extrait en tête du dernier étage de détente est envoyé en bas de l'avant-dernier étage de détente en même temps que la vapeur d'eau avec laquelle fonctionne la trompe à vapeur d'eau.

**Claims**

1. A process for the removal of $CO_2$ and/or $H_2S$ from gases containing $CO_2$ and/or $H_2S$, in which

a) the gases containing $CO_2$ and/or $H_2S$ are treated in an absorption stage at from 40 to 100°C with an aqueous absorption liquid containing from 20 to 70% by weight of methyldiethanolamine,

b) the treated gases are taken off at the top of the absorption stage,

c) the aqueous absorption liquid laden with $CO_2$ and/or $H_2S$ is taken off at the bottom of the absorption stage and, for regeneration, is let down in two or more let-down stages, and the final let-down stage is operated under reduced pressure,

d) to compensate for the water losses resulting from water present in the gas streams taken off at the top of the absorption stage and the let-down stages, an amount of steam corresponding to the water loss is fed in at the bottom of the penultimate let-down stage, and

e) the regenerated absorption liquid is recycled to the absorption stage.

2. A process as claimed in claim 1, wherein the reduced pressure in the final let-down stage is produced by means of a steam ejector, and the gas taken off at the top of the final let-down stage is fed, together with the stream with which the steam ejector is operated, to the bottom of the penultimate let-down stage.

FIG.1

FIG.2